# EUROPEAN PATENT SPECIFICATION

(11) **EP 2 695 605 B1**
(45) Date of publication and mention of the grant of the patent: **03.05.2017**
(21) Application number: 12179521.5
(22) Date of filing: 07.08.2012
(51) Int. Cl.: A61K 9/08, A61K 31/195, A61K 47/18

(54) **Tranexamic acid composition**
Tranexamsäurezusammensetzung
Composition d'acide tranexamique

(43) Date of publication of application: 12.02.2014
(73) Proprietor: Parapharm Development Ltd., Windsor Berkshire SL4 1RU (GB)
(72) Inventor: Velada, José Luis, 3826 BA Amersfoort (NL); Backers, Onne Peter Hilbert, 6813 DR Arnhem (NL); Doshi, Hiteshkumar Anilkant, 400080 Mumbai (IN); Shinde, Vicky, 400080 Mumbai (IN); Tendulkar, Anjali Rajesh, 400080 Mumbai (IN); Shah, Avni, 400080 Mumbai (IN)
(74) Representative: Swarte, Veronica Regina

(56) References cited:
- EP-A2- 1 057 490
- WO-A1-2011/078700
- CHRISTMAS S E ET AL: "The effects of immunosuppression and anticoagulation on fibrin deposition and swelling in rat cardiac allografts", CLINICAL IMMUNOLOGY AND IMMUNOPATHOLOGY, SAN DIEGO, CA, US, vol. 42, no. 1, 1 January 1987 (1987-01-01), pages 18-26, XP026198096, ISSN: 0090-1229, DOI: 10.1016/0090-1229(87)90169-3 [retrieved on 1987-01-01]
- BURT T ET AL: "Dental procedures in patients with bleeding disorders and the cost effectiveness of 'make your own' tranexamic acid mouthwash", BRITISH JOURNAL OF HAEMATOLOGY, vol. 157, no. Suppl. 1, Sp. Iss. SI, April 2012 (2012-04), page 38, XP002688504, & 52ND ANNUAL SCIENTIFIC MEETING OF THE BRITISH-SOCIETY-FOR-HAEMATOLOGY; GLASGOW, UK; APRIL 16 -18, 2012 ISSN: 0007-1048

## Description

### Field of the invention

The invention is in the field of preparations, in particular pharmaceutical compositions, comprising tranexamic acid.

### Background of the invention

Tranexamic acid (*trans*-4-(aminomethyl)cyclohexanecarboxylic acid) is an antifibrinolytic agent, widely used for the prevention and control of bleeding. Tranexamic acid compositions have been found to be effective in controlling bleeding in patients suffering from trauma, menorrhagia and in patients who had undergone orthopaedic or heart surgeries. Aqueous solutions of tranexamic acid have been used to prepare parenteral antifibrinolytic compositions and to prepare oral compositions that prevent bleeding after dental extraction surgeries. A major problem of tranexamic acid containing compositions is that they are unstable, undergo colouration and develop an unpleasant taste, upon storage. No storage stable tranexamic acid liquid oral compositions are currently commercially available. Tablet compositions that are commercially available for instant preparation of tranexamic acid solutions also do not render storage stable solutions.

The mechanism underlying instability and colouration of tranexamic acid compositions remain largely unclear. Nevertheless, various efforts have been made in the past, to prevent instability and colouration of tranexamic acid containing compositions. These include addition of stabilizing agents such as antioxidants or exclusion of agents causative of colouration.

JP 3279313 discloses a method for prevention of colouration of a tranexamic acid containing cosmetic skin preparation by addition of sodium hydrogen sulphite as a stabilizing agent. WO 2010/027010 discloses solid tranexamic acid containing solid preparations for oral administration wherein colouration is prevented by addition of an antioxidant such as ascorbic acid. JP 2006182655 discloses tranexamic acid solutions where colouration is prevented by reducing the quantity of certain sweetening agents that are causative of colouration.

T Burt et al., British Journal of Haematology, Vol. 157, Suppl. 1, abstract 94, discusses the short shelf life of tranexamic acid mouthwash.

### Summary of the invention

There is an ongoing need for improved tranexamic acid containing compositions, preferably with respect to improvements of stability upon storage, more preferably with respect to improvements of stability regarding colouration upon storage, preferably with respect to liquid tranexamic acid containing compositions. Preferably the aim is to provide tranexamic acid containing compositions that are resistant to colouration even in the presence of agents that are causative of colouration such as sweetening agents. Surprisingly it was found that certain pH buffering agents, in addition to maintaining the pH of tranexamic acid compositions in the desired range, preferably in the range of 6 to 8, are capable of imparting stability and resistance to colouration of tranexamic acid solutions over a prolonged period of time.

Accordingly, the invention provides an oral rinse composition comprising tranexamic acid as an active ingredient and a pH buffering agent selected from Tris and glycine, wherein the weight ratio tranexamic acid to buffering agent is in the range of 100:1 1 to 2:1. Conventionally used tranexamic acid containing compositions display a brown colour over a period of time, which can be analytically evidenced by significant absorbance for a radiation having a wavelength of 420 nm. It has now been found that, upon storage, unlike conventional tranexamic acid containing compositions, a composition comprising tranexamic acid in the presence of certain pH buffering agent(s) does not display the characteristic brown colour. In other words, according to the invention, a composition comprising tranexamic acid is provided that is stable, for example upon storage for at least 6 months, and is colourless and has negligible absorbance at a wavelength of 420 nm. Thus, the pH buffering agent(s) used in the composition according to the invention impart stability to the composition, and resistance to colouration. The pH buffering agent used in the composition according to the invention impart stability to the composition even in the absence of conventionally used stabilising agents such as antioxidants or even in the presence of colouration promoting sweetening agents. Typically, the composition of the invention, when in liquid form, has an absorbance of less than 0.03 for radiation having wavelength of 420 nm using a path length of 1 cm.

### Detailed description of the invention

Accordingly, the invention provides buffered tranexamic acid containing compositions that are storage-stable and are resistant to coloration. The pH buffering agent is selected from 2-amino-2-hydroxymethyl-propane-1,3-diol and 2-aminoacetic acid. The compound 2-amino-2-hydroxymethyl-propane-1,3-diol is also known as tris(hydroxymethyl)aminomethane or THAM or TRIS and the compound 2-aminoacetic acid is also known as glycine.

It is to be understood that the pH buffering agent may, depending on the pH, be deprotonated at the CO₂H group and/or protonated at the NH₂ group and hence can be provided as a pharmaceutically acceptable salt.

The tranexamic acid containing composition of the invention comprises a pH buffering agent selected from Tris and glycine. The pH buffering agent present in the composition of the invention is preferably used in a concentration that imparts the desired pH in the range of 6 to 8. Preferably, the composition according to the invention is a liquid having a pH in the range of about 6 to about 8. More preferably, the pH buffering agent present in the composition of the invention imparts a pH in the range of 6.4 - 8.0, to the composition. Most preferably, the pH of the liquid composition is about 6.5.

In case the composition according to the invention is a liquid, the pH buffering agent is typically present at a concentration of at least 0.01 M. Preferably, the pH buffering agent is present at a concentration in the range of 0.01 M to 0.1 M, more preferably at a concentration in the range of 0.01 M to 0.05 M.

Preferably, the pH buffering agent is present in a concentration in the range of 0.05 to 3.5 wt.% based on the total weight of the composition. More preferably, the pH buffering agent is used in a concentration in the range of 0.12 to 0.6 wt.% based on the weight of the composition.

The composition of the invention preferably comprises 0.5 wt.% to 25 wt.% tranexamic acid, more preferably 2 wt.% to 10 wt.%, most preferably about 5 wt.%.

The composition of the invention comprises tranexamic acid and the pH buffering agent in a weight ratio of tranexamic acid:pH buffering agent between 100:1 and 2:1, more preferably between 50:1 and 4:1, even more preferably between 40:1 and 10:1.

The present composition is stable upon storage, preferably at room temperature. Preferably the present composition is stable upon storage for at least 3 months, more preferably at least 6 months at room temperature. Preferably, the stability is upon storage at 20°C. Stable upon storage means that the composition, when in liquid form, has an absorbance of less than 0.03 for a radiation having wavelength 420 nm using a path length of 1 cm. Preferably the composition according to the invention is in liquid form and has an absorbance of less than 0.03 for a radiation having wavelength 420 nm.

In a further embodiment, the composition according to the invention further comprises a sweetening agent. It is found that the pH buffering agent in the tranexamic acid containing composition of the invention is capable of resisting colouration of the composition even in the presence of sweetening agent(s).
Suitable sweetening agents for pharmaceutical compositions, preferably for liquid compositions, are known in the art. Preferably the sweetening agent is selected from the group consisting of monosaccharides, disaccharides, polysaccharides, sugar alcohols, aspartame, acesulfame potassium, cyclamate, saccharin, saccharin sodium, sucralose or mixtures thereof. More preferably the sweetening agent is selected from the group consisting of xylose, ribose, glucose, mannose, galactose, fructose, dextrose, sucrose, maltose, partially hydrolyzed starch or corn syrup solids, sorbitol, xylitol, mannitol, pentaerythritol, glycerine, aspartame, acesulfame potassium, cyclamate, saccharin, saccharin sodium, sucralose and combinations thereof. Preferably the composition according to the invention comprises an artificial, i.e. non-sugar, sweetening agent, more preferably the composition according to the invention comprises sucralose.

The amount of sweetener used in the composition of the invention will vary depending on the nature of the sweetener and the degree of palatability desired for the composition. Typically, the sweetening agent used in the composition of the invention is used in an amount ranging from 0.02 to 1 wt.% based on the weight of the composition when an artificial sweetening agent is used and from 5 to 30 wt.% based on the weight of the composition when a sugar sweetening agent is used. The composition according to the invention is for oral administration, i.e. it is administered orally. The composition according to the invention is an oral rinse composition.

In addition to pH buffering agent(s) and sweetening agent(s), the composition of the invention can contain other customary pharmaceutical ingredients. The pharmaceutical ingredients that can be present in the composition of the invention include, but are not limited to, optional flavouring agents, preservatives, further pH adjusting agents and the like. Flavouring agents that can be added to the composition of the invention are those known in the pharmaceutical art. Typically, the flavouring agent is selected from synthetic flavour oils and/or naturally derived oils from plants, flowers, leaves, other natural flavours, natural fruit flavours, artificial flavours, artificial fruit flavours, or combinations thereof. Naturally derived oils, other natural flavours, synthetic flavours or combinations thereof include, but are not limited to, mint (such as peppermint or spearmint), menthol, cinnamon, vanilla, artificial vanilla, chocolate or artificial chocolate. Natural fruit flavours, artificial fruit flavours or combination thereof include, but are not limited to, cherry, grape, orange, strawberry or lemon. In one embodiment, the flavouring agent used in the composition of the invention is present in a range of about 0.01 to about 0.20 wt.% based on the weight of the composition. Advantageously, a flavouring agent is present in a range of about 0.10 to about 0.20 wt.% based on the weight of the composition.

Preservatives useful in the composition of the invention include, but are not limited to, sodium benzoate, potassium sorbate, EDTA or salts thereof, parabens (such as methyl, ethyl, propyl and butyl p-hydroxybenzoic acids esters or mixtures thereof) or mixtures thereof. It may be noted that the preservatives listed above are exemplary, but must be evaluated on an empirical basis, in each composition, to ensure the compatibility and efficacy. Methods for evaluating the efficacy of preservatives in pharmaceutical compositions are known to those skilled in the art. Advantageously, methylparaben, propylparaben, sodium benzoate, or a mixture thereof is used as a preservative. Preservatives can be present in amounts of up to about 1 gram per 100 ml, or up to 1 wt.%. Advantageously, an individual preservative can be present in an amount in the range of from about 0.01 to about 1.0 wt.% based on the weight of the composition.

More advantageously, a preservative such as methylparaben, propylparaben, butyl-paraben or a mixture thereof is present in a range of from about 0.02 to about 0.5 wt.% based on the weight of the composition. Still more advantageously, about 0.02 to 0.2 wt.% based on the weight of the composition of a preservative selected from methylparaben, propylparaben and mixtures thereof is present in the composition of the invention.

The composition of the present invention can additionally contain humectant(s) and/or surfactant(s). The humectant(s) in the composition of the invention may range from 10 to 40 wt.% based on the weight of the composition. Humectant(s) that can be present in the composition of the invention include, but are not limited to, sorbitol, glycerine, ethylene glycol, propylene glycol, 1,3-butylene glycol, polyethylene glycol, polypropylene glycol, xylitol, maltitol, lactitol, and mixtures thereof. The composition of the invention can also contain surfactants, in particular components belonging to the class of conventional pharmaceutically applicable surfactants. When a surfactant is employed, the amount present in the compositions of the invention will vary depending on the particular surfactant chosen, the particular mode of administration (e.g. drop or spray) and the effect desired. In general, however, the amount present is in the range of 0.01 to about 0.5 wt.%, preferably about 0.05 to 0.2 wt.% and more preferably about 0.1 wt.% based on the weight of the composition.

In a preferred embodiment, the composition of the invention is a liquid composition. The composition of the invention is an oral rinse. Further, the composition of the invention can be suitable to be administered to patients who have undergone dental extraction, to patients in association with surgery or other trauma, to patients suffering from menorrhagia or to patients receiving anticoagulant therapy. The dosage of administration is determined as per the requirement under each situation and can be increased or decreased according to the degree of a particular condition.

The invention is further illustrated by way of the following non-limiting example.

### EXAMPLE: Comparative study of Absorbance of tranexamic acid compositions prepared by using different pH buffering agents

Absorbance measurements were carried out in a Shimadzu 1800 UV spectrophotometer, using cuvettes with a path length of 1 cm. The measurements were carried out for the samples before and after accelerated stability test at 60°C for 15 days. Tranexamic acid used in the composition was obtained from Hunan Donting, China. The buffering agents used in the composition of the invention were obtained from Merck.

Aqueous tranexamic acid oral compositions were prepared by mixing thoroughly the following ingredients under stirring
Tranexamic acid (5 wt%)
Xylitol (20 wt%)
Methylparaben (0.15 wt%)
Propylparaben (0.02 wt%)
pH buffering agent (as displayed in table 1)
Peppermint flavour (0.20 wt%)
1M HCl q.s (for adjusting pH to the value as indicated in table 1)
Purified water q.s (up to 100 wt%)

For each experiment, a pH buffering agent, as displayed in table 1, in the concentration as displayed in table 1 was added and the pH was determined. Absorbance measurements were carried out in samples before and after these were subjected to accelerated stability test at 60°C for 15 days. The results of the measurement are displayed in table 2.

**Table 1: Compositions used in the accelerated stability test (results see table 2)**

| No. | pH buffering agent | amount pH buffering agent | | | amount tranexamic acid in wt% [b] | pH |
|---|---|---|---|---|---|---|
| | | wt% [a] | wt% [b] | conc [c] | | |
| 1 | Phosphate [d] | 1.5512 | 5.805 | 0.05 | 18.713 | 6.73 |
| 2 | Isoleucine | 0.6558 | 2.539 | 0.05 | 19.362 | 6.36 |
| 3 | Glycine | 0.3752 | 1.468 | 0.05 | 19.574 | 6.27 |
| 4 | Tris | 0.1211 | 0.479 | 0.01 | 19.771 | 7.20 |
| 5 | Tris | 0.2422 | 0.953 | 0.02 | 19.677 | 7.23 |
| 6 | Tris | 0.6057 | 2.350 | 0.05 | 19.400 | 7.24 |

| | | | | | | |
|---|---|---|---|---|---|---|
| [a] based on total weight of the composition; [b] based on dry weight of the composition; [c] concentration in mol/L (M); [d] phosphate buffering agent imparting a pH of 6.73 was prepared using an equimolar combination of potassium dihydrogen phosphate (0.6804 wt% on total weight; 2.549 wt% on dry weight) and dipotassium hydrogen phosphate (0.8708 wt% on total weight; 3.259 wt% on total weight). | | | | | | |

**Table 2: Comparison of absorbance of the tranexamic acid compositions of table 1, before and after the accelerated stability test at 60°C for 15 days**

| No. [a] | pH buffering agent | Absorbance (at 420 nm) after T days of storage (T = number of days) | |
|---|---|---|---|
| | | T = 0 | T = 15 days |
| 1 | Phosphate | 0.0198 | 0.3749 |
| 2 | Isoleucine | 0.0034 | 0.7354 |
| 3 | Glycine | 0.0178 | 0.0282 |
| 4 | Tris (0.01 M) | 0.0022 | 0.0217 |
| 5 | Tris (0.02 M) | 0.0046 | 0.0181 |
| 6 | Tris (0.05 M) | 0.0058 | 0.0287 |

| | | | |
|---|---|---|---|
| [a] corresponding to table 1. | | | |

From table 2, it is clear that tranexamic acid compositions that contain the conventional phosphate buffer (experiment No. 1) displayed significant absorbance after accelerated stability test at 60°C for 15 days (a 19-fold increase in absorbance at 420 nm). Similarly, when isoleucine was used as buffering agent (experiment No. 2), the tranexamic acid containing solutions displayed significant absorbance in the sample after the stability test (> 200-fold increase in absorbance at 420 nm). On the contrary, the tranexamic acid containing solutions comprising glycine or tris as buffering agent (experiment Nos. 3 to 6) display negligible absorbance, below 0.03, at 420 nm, after accelerated stability test (< 10-fold increase in absorbance at 420 nm). The measured absorbances of the samples were in accordance with their visual appearance. Thus, while the samples of experiment Nos. 1 and 2 that contain phosphate as buffering agent and isoleucine as buffering agent respectively appeared brown to dark brown coloured after accelerated stability test at 60°C for 15 days, the corresponding samples of experiment Nos. 3 to 6, containing either glycine or tris as buffering agent, appeared colourless upon visual examination after the stability test. The colouration caused upon accelerated stability test of samples of experiment Nos. 1 and 2 signifies instability presumably due to chemical degradation of the corresponding tranexamic acid compositions. The absence of colour and of significant absorbance in the samples containing glycine or tris as buffering agent signifies high stability and resistance to degradation of the tranexamic acid compositions that contains these buffers.

The advantageous stability and consequent resistance to colouration of tranexamic acid containing compositions of the invention enables commercial utilization of the compositions for various applications including usage as oral rinse compositions. The surprising stability of the tranexamic acid composition achieved by using pH buffering agent(s) according to the invention , results in improvement of shelf life of the composition and eliminates the requirement of additional stabilizing agents. Further, the stability against degradation and resistance to colouration displayed by the tranexamic compositions of the invention aids formulation development by allowing minimal use of excipients and enables as well substantial savings in cost.

The above description is illustrative only and is not limiting. The present invention is defined by the claims that follow.

## Claims

1. An oral rinse composition comprising tranexamic acid as an active ingredient and a pH buffering agent selected from Tris and glycine, wherein the weight ratio tranexamic acid to buffering agent is in the range of 100:1 to 2:1.

2. The oral rinse composition according to claim 1, which is liquid.

3. The oral rinse composition according to claim 2, which has pH in the range of 6 to 8.

4. The oral rinse composition according to claim 2 or 3, which has an absorbance of less than 0.03 for a radiation having a wavelength of 420 nm.

5. The oral rinse composition according to any one of claims 1 to 4, further comprising a sweetening agent.

6. The oral rinse composition according to claim 5, wherein the sweetening agent is selected from the group consisting of monosaccharides, disaccharides, polysaccharides, sugar alcohols, aspartame, acesulfame potassium, cyclamate, saccharin, saccharin sodium, sucralose and mixtures thereof.

7. The oral rinse composition according to any one of claims 1 to 6, which comprises 2 wt.% to 10 wt.% tranexamic acid.

## Patentansprüche

1. Mundspülungszusammensetzung, die Tranexamsäure als wirksamen Bestandteil und ein aus Tris und Glycin ausgewähltes pH-Puffermittel umfasst, wobei das Gewichtsverhältnis von Tranexamsäure zu Puffermittel im Bereich von 1:100 bis 2:1 ist.

2. Mundspülungszusammensetzung gemäß Anspruch 1, die flüssig ist.

3. Mundspülungszusammensetzung gemäß Anspruch 2, die einen pH im Bereich von 6 bis 8 hat.

4. Mundspülungszusammensetzung gemäß Anspruch 2 oder 3, die eine Extinktion von weniger als 0,03 für Strahlung mit einer Wellenlänge von 420 nm hat.

5. Mundspülungszusammensetzung gemäß einem der Ansprüche 1 bis 4, die weiterhin ein Süßungsmittel umfasst.

6. Mundspülungszusammensetzung gemäß Anspruch 5, worin das Süßungsmittel aus der Gruppe ausgewählt ist, die aus Monosacchariden, Disacchariden, Polysacchariden, Zuckeralkoholen, Aspartam, Acesulfam-Kalium, Cyclamat, Saccharin, Saccharin-Natrium, Sucralose und Mischungen daraus besteht.

7. Mundspülungszusammensetzung gemäß einem der Ansprüche 1 bis 6, die 2 bis 10 Gew.-% Tranexamsäure umfasst.

## Revendications

1. Composition orale de rinçage comprenant de l'acide tranexamique à titre d'ingrédient actif et un agent tampon de pH choisi parmi Tris et glycine, dans laquelle le ratio en poids de l'acide tranexamique par rapport à l'agent de tampon pH est dans la gamme de 100:1 à 2:1.

2. Composition orale de rinçage selon la revendication 1, ladite composition étant liquide.

3. Composition orale de rinçage selon la revendication 2, ladite composition ayant un pH dans la gamme de 6 à 8.

4. Composition orale de rinçage selon la revendication 2 ou 3, ladite composition ayant une absorbance de moins de 0,03 pour une radiation ayant une longueur d'onde de 420 nm.

5. Composition orale de rinçage selon l'une quelconque des revendications 1 à 4, comprenant en outre un agent édulcorant.

6. Composition orale de rinçage selon la revendication 5, dans laquelle l'agent édulcorant est choisi parmi le groupe constitué par des monosaccharides, des disaccharides, des polysaccharides, des alcools de sucre, l'aspartame, l'acésulfame de potassium, le cyclamate, la saccharine, la saccharine de sodium, le sucralose et des mélanges de ceux-ci.

7. Composition orale de rinçage selon l'une quelconque des revendications 1 à 6, ladite composition comprenant de 2 à 10 % en poids d'acide tranexamique.
